# EUROPÄISCHE PATENTSCHRIFT

(11) **EP 3 116 451 B1**
(45) Veröffentlichungstag und Bekanntmachung des Hinweises auf die Patenterteilung: **10.04.2019**
(21) Anmeldenummer: 15707911.2
(22) Anmeldetag: 26.02.2015
(51) Int. Cl.: A61F 2/46, A61F 2/38, A61F 2/30

(54) **VERBINDUNGSEINRICHTUNG ZUR VERBINDUNG ZWEIER PROTHESENTEILE UND SATZ MIT EINER DERARTIGEN VERBINDUNGSEINRICHTUNG UND ZWEI PROTHESENTEILEN**
CONNECTION DEVICE FOR CONNECTING TWO PROSTHESIS PARTS, AND KIT WITH ONE SUCH CONNECTION DEVICE AND TWO PROSTHESIS PARTS
DISPOSITIF DE LIAISON DESTINÉ À LA LIAISON DE DEUX PARTIES DE PROTHÈSE ET ENSEMBLE COMPOSÉ D'UN TEL DISPOSITIF DE LIAISON ET DE DEUX PARTIES DE PROTHÈSE

(30) Priorität: 10.03.2014 DE 102014204326
(43) Veröffentlichungstag der Anmeldung: 18.01.2017
(73) Patentinhaber: Waldemar Link GmbH & Co. KG, 22339 Hamburg (DE)
(72) Erfinder: ASMUS, Dörte, 20249 Hamburg (DE); DMUSCHEWSKY, Klaus, 22397 Hamburg (DE)
(74) Vertreter: Hoffmann Eitle
(86) Internationale Anmeldenummer: PCT/EP2015/054046
(87) Internationale Veröffentlichungsnummer: WO 2015/135762

(56) Entgegenhaltungen:
- EP-A1- 0 923 916
- DE-A1-102012 001 464
- FR-A1- 2 848 412

## Beschreibung

### Technisches Gebiet

Die Erfindung betrifft eine Verbindungseinrichtung zur Verbindung eines ersten und zweiten Prothesenteils, insbesondere einer Tibia- und einer Femur-Probeprothese zur probeweisen Anordnung an den entsprechenden Knochen. Das erste Verbindungselement dient zur Anbringung an dem ersten Prothesenteil und das zweite Verbindungselement dient zur Aufnahme eines Abschnitts des zweiten Prothesenteils. Dabei ist das zweite Verbindungselement relativ zu dem ersten Verbindungselement verschiebbar.

### Stand der Technik

Verbindungseinrichtungen der eingangsgenannten Art finden bei Gelenksprothesen breite Anwendung. Insbesondere bei Kniegelenksprothesen kann die Verbindungseinrichtung die Kondylen des Femurs mit einer Lagerfläche der Tibia verbinden.

Bereits während einer Operation soll festgestellt werden, ob die Prothesen hinsichtlich Rotation, Flexion und Hyperextension geeignet sind. Dazu können Probeprothesen verwendet werden, die vorübergehend während der Operation vor Einsetzen der endgültigen Prothesen eingesetzt werden.

Diese Probeprothesen werden herkömmlicherweise durch Verschrauben zweier Klemmbacken verbunden, welche einen Steg zwischen den Kondylen des Femurs einklemmen. Bei Schraubverbindungen besteht jedoch der Nachteil, dass die Befestigung aufwendig ist und während der Operation nur schwer einhändigt durchzuführen ist.

Eine andere Möglichkeit der Verbindung ist in der EP 0 923 916 A1 offenbart, bei der ein Zapfen mit einem umlaufenden Vorsprung vorgesehen ist, in den der Steg zwischen den Kondylen des Femurs aufgenommen werden kann. Der Zapfen ist in die Tibia-Prothese hineinverschiebbar.

Derartige Verbindungseinrichtungen haben sich bewährt. Allerdings werden sowohl hinsichtlich der Verlässlichkeit bezüglich ungewollter Lösbarkeit, der stabilen Führung durch die Verbindungskomponenten als auch der Handhabbarkeit an derartige Verbindungseinrichtungen erhöhte Anforderungen gestellt.

### Darstellung der Erfindung

Es ist somit Aufgabe der vorliegenden Erfindung, bei einfacher Konstruktion die Handhabbarkeit und die Verlässlichkeit einer Verbindungseinrichtung zur Verbindung zweier Prothesenteile zu verbessern. Es soll insbesondere eine Verbindungseinrichtung geschaffen werden, die während einer Operation eine leichtgängige und gut handhabbare Verbindung zwischen zwei Prothesenteilen ermöglicht. Des Weiteren soll die Verbindungseinrichtung eine definierte Lagerung und präzise Führung der beiden Probeprothesen relativ zueinander ermöglichen.

Diese Aufgabe wird erfindungsgemäß durch die Verbindungseinrichtung mit dem Merkmal von Anspruch 1 gelöst. Demzufolge weist die Verbindungseinrichtung ein erstes Verbindungselement, das als Führung dienende Innenwände und ein Gegenstück aufweist, zur Anbringung an dem ersten Prothesenteil und ein zweites Verbindungselement auf, das zumindest abschnittsweise von den Innenwänden des ersten Verbindungselement umschlossen ist, entlang den Innenwänden relativ zu dem ersten Verbindungselement in eine Verschiebungsrichtung verschiebbar ist und eine Nut zur Aufnahme eines Abschnitts des zweiten Prothesenteils aufweist. Dabei ist das zweite Verbindungselement in die Verschiebungsrichtung relativ zum ersten Verbindungselement in eine offene Position, in der das zweite Verbindungselement zumindest abschnittsweise aus dem ersten Verbindungselement herausgeschoben ist und die Nut des zweiten Verbindungselement zumindest abschnittsweise derart frei liegt, dass der Abschnitt des zweiten Prothesenteils in die Nut eingeführt und aus der Nut herausgeführt werden kann, und in eine geschlossene Position verschiebbar, in der das zweite Verbindungselement zumindest abschnittsweise in das erste Verbindungselement hineingeschoben ist und das Gegenstück des ersten Verbindungselements der Nut des zweiten Verbindungselements derart verschließend gegenüberliegt, dass ein Austreten des in der Nut aufgenommenen Abschnitts des zweiten Prothesenteils durch das Gegenstück des ersten Verbindungselements aus der Nut verhindert wird.

Die erfindungsgemäße Verbindungseinrichtung ermöglicht eine stabile Führung des zweiten Verbindungselements durch die Führung in dem ersten Verbindungselement mittels der Innenwände des ersten Verbindungselements. Darüber hinaus wird durch die erfindungsgemäße Verbindungseinrichtung die Probeprothese hinsichtlich Rotation, Flexion und Hyperextension begrenzt. Der ROM ("Range of Motion" oder "Bewegungsumfang") entspricht dem der später dauerhaft einzusetzenden Implantate und ermöglicht eine Rotation von ± 15°, eine Flexion von 120° und eine Hyperextension von 2,5°. Gleichzeitig ist es durch die definierte Verschiebbarkeit in zwei Positionen möglich, das zweite Verbindungselement in eine Position zu verschieben, in der der Abschnitt des zweiten Prothesenteils in die Nut eingeführt bzw. aus der Nut herausgeführt werden kann, und in eine dazu unterschiedliche geschlossene Position zu verschieben, in der die Nut dem Gegenstück derart gegenüberliegt, dass die Nut im Wesentlichen geschlossen ist und der Abschnitt des zweiten Prothesenteils aus der Nut nicht austreten kann. Somit kann sichergestellt werden, dass eine zuverlässige Verbindung zwischen dem ersten und dem zweiten Prothesenteil vorliegt, solange die Verbindungseinrichtung bzw. das zweite Verbindungselement in der geschlossenen Position ist. Lediglich in der offenen Position kann eine Trennung zwischen dem ersten und dem zweiten Prothesenteil erfolgen. Um zwischen den beiden Positionen zu wechseln, muss lediglich das zweite Verbindungselement relativ zu dem ersten Verbindungselement verschoben werden.

Es ist möglich, verschiedene Größen der erfindungsgemäßen Verbindungseinrichtung entsprechend den Größen der Probeprothesen bzw. der Implantate zu verwenden. Insbesondere für kleine Größen sollte eine spezielle Verbindungseinrichtung mit kleineren Abmessungen verwendet werden.

Der Erfindung liegt der Gedanke zugrunde, eine Verbindungseinrichtung zur verlässlichen und einfach handhabbaren Verbindung zwischen einem ersten und zweiten Prothesenteil zu schaffen, wobei durch Verschieben des zweiten Verbindungselements durch eine stabile und definierte Führung in einer Position eine Aufnahme bzw. Entnahme des zweiten Prothesenteils möglich ist und einer dazu unterschiedlichen Position der Abschnitt des zweiten Prothesenteils sicher aufgenommen ist. Der Positionswechsel erfolgt durch Verschieben des ersten und zweiten Verbindungselements zueinander, sodass eine "schraubenfreie" Verbindung zwischen zwei Probeprothesen ermöglicht wird. Somit sind weder eine Schraube noch ein Gewinde beim Herstellen bzw. Lösen der Verbindung beteiligt.

Das Gegenstück, das der Nut zum Verschließen der Nut gegenüberliegt, kann insbesondere auch ein Teil der Innenwände sein, die als Führung dienen.

Das zweite Verbindungselement ist mit anderen Worten gleitbar in dem ersten Verbindungselement gelagert und umfangsmäßig von dem ersten Verbindungselement bzw. den Innenwänden des ersten Verbindungselements umschlossen.

Darüber hinaus sind unter der Verschiebungsrichtung die Richtungen zu verstehen, in die die Relativbewegung zwischen dem ersten und zweiten Verbindungselement während des Übergangs von der offenen zur geschlossenen bzw. von der geschlossenen zu der offenen Position hin erfolgt. Die Verschiebungsrichtung zur offenen Position hin ist der Verschiebungsrichtung zur geschlossenen Position hin entgegengesetzt.

Besonders vorteilhafte Weiterbildungen der Erfindung sind in den abhängigen Ansprüchen angegeben.

Vorzugsweise ist das Gegenstück als Vorsprung ausgebildet, der in der Verbindungsrichtung zu der offenen Position hin von dem ersten Verbindungselement vorsteht. Mit anderen Worten kann das Gegenstück nach oben hin vorstehen. Dadurch kann die Nut vorteilhaft verschlossen bzw. abgedeckt werden, wobei gleichzeitig die Distanz zwischen der offenen Position und der geschlossenen Position, die in Verschiebungsrichtung zurückgelegt werden muss, reduziert ist.

Die Nut verläuft insbesondere im Wesentlichen senkrecht zu der Verschiebungsrichtung. Weiter vorzugsweise definiert die Nut eine Öffnung, die im Wesentlichen senkrecht zur Verschiebungsrichtung geöffnet ist. Mit anderen Worten zeigt die Öffnung der Nut senkrecht zur Verschiebungsrichtung. Somit kann eine von gegenüberliegenden Wänden der Nut beim Verschieben des zweiten Verbindungselements in die Verschiebungsrichtung den Abschnitt des zweiten Prothesenteils, der in der Nut aufgenommen ist, in die Verschiebungsrichtung mitführen. Durch die derartige Orientierung der Nut bzw. der Öffnung ist sowohl ein einfaches Heraus- und Hereinführen eines aufgenommenen Abschnitts als auch ein Verschieben des zweiten Verbindungselements möglich, ohne dass der aufgenommene Abschnitt zum Beispiel unbeabsichtigt herausrutscht. Das Gegenstück kann als derartiger Vorsprung ausgebildet sein, dass er als im Wesentlichen vertikaler Plattenabschnitt von dem ersten Verbindungselement nach oben hin vorsteht, sodass das vertikale Gegenstück gut die vertikal geöffnete Nut abdecken kann.

Das erste Verbindungselement kann ein Federelement, insbesondere eine Federlasche, zum Drücken gegen das zweite Verbindungselement aufweisen, wodurch ein ungewolltes Verschieben des zweiten Verbindungselements in die Verschiebungsrichtung verhindert wird. Die Federlasche übt folglich eine Reibungskraft aus, wenn sie gegen das zweite Verbindungselement drückt und dieses insbesondere zur offenen Position hin verschoben werden soll. Bei einem beabsichtigten Verschieben kann diese Kraft leicht überwunden werden; bei einem unbeabsichtigten Verschieben hingegen kann eine Verschiebung des zweiten Verbindungselements relativ zum ersten Verbindungselement verhindert werden.

Es wird bevorzugt, dass das Federelement in Bezug auf das Gegenstück in der Verschiebungsrichtung zu der geschlossenen Position hin angeordnet ist, das Federelement also unter dem Gegenstück positioniert ist. Es bietet sich insbesondere an, dass sich eine Federlasche von dem Gegenstück zu dem untersten Abschnitt des ersten Verbindungselements erstreckt.

Vorzugsweise weist das zweite Verbindungselement einen Stufenabschnitt auf und das erste Verbindungselement einen komplementären Anschlag, sodass die maximale Verschiebung des zweiten Verbindungselements relativ zu dem ersten Verbindungselement in der offenen Position durch ein Anstoßen des Stufenabschnitts und des Anschlags aneinander begrenzt ist. Somit kann ein vollständiges, ungewolltes Lösen des zweiten Verbindungselements von dem ersten Verbindungselement verhindert werden.

Erfindungsgemäß ist ein Satz mit einer erfindungsgemäßen Verbindungseinrichtung, einem ersten Prothesenteil und einem zweiten Prothesenteil vorgesehen. Vorzugsweise ist der Abschnitt des zweiten Prothesenteils, der in dem zweiten Verbindungselement aufgenommen werden soll, als Achse quer zur Verschiebungsrichtung und mit einem Querschnitt ausgebildet, der im Wesentlichen dem Querschnitt der Freifläche der Nut des zweiten Verbindungselements entspricht. Dadurch kann der Abschnitt des zweiten Prothesenteils im Wesentlichen ohne Spiel in dem zweiten Verbindungselement aufgenommen werden, was insbesondere die Führung bzw. die Lagerung und Ausrichtung des ersten Prothesenteils relativ zu dem zweiten Prothesenteil genau definiert.

Vorzugsweise weist das erste Prothesenteil eine Führung des ersten Verbindungselements in eine Richtung senkrecht zur Verschiebungsrichtung zur Anbringung des ersten Verbindungselements an dem ersten Prothesenteil auf. Dadurch kann auch das erste Verbindungselement leicht an der Tibia-Probenprothese befestigt werden, wobei die Position des ersten Verbindungselements in die Verschiebungsrichtung, also senkrecht zur Führung im ersten Prothesenteil, festgelegt ist, sodass bei Verschiebung des zweiten Verbindungselements relativ zum ersten Verbindungselement in die Verschiebungsrichtung das erste Verbindungselement in der ersten Verbindungsrichtung definiert festgehalten wird.

Das erste als auch das zweite Prothesenteil bestehen vorzugsweise aus einem Metall, einer Metalllegierung, Kunststoff und/oder Keramik.

Weitere Merkmale und Vorteile der Erfindung werden anhand der nachfolgenden ausführlichen Beschreibung noch näher ersichtlich werden.

### Kurze Beschreibung der Zeichnungen

- Fig.1(a): zeigt ein erstes Verbindungselement und ein zweites Verbindungselement gemäß der vorliegenden Erfindung in der geschlossenen Position;
- Fig.1(b): zeigt ein erstes Verbindungselement und ein zweites Verbindungselement gemäß der vorliegenden Erfindung in der offenen Position;
- Fig.1(c): zeigt ein zweites Verbindungselement gemäß der vorliegenden Erfindung;
- Fig.1(d): zeigt ein erstes Verbindungselement gemäß der vorliegenden Erfindung in einer Vorderansicht;
- Fig.1(e): zeigt ein erstes Verbindungselement gemäß der vorliegenden Erfindung in einer Rückansicht.
- Fig.2: zeigt einen Satz mit der Verbindungseinrichtung, einer Tibia-Probeprothese und einer Femur-Probeprothese gemäß der vorliegenden Erfindung.

Ausführliche Beschreibung einer bevorzugten Ausführungsform

Eine bevorzugte Ausführungsform der vorliegenden Erfindung wird nachfolgend ausführlich unter Bezugnahme auf die begleitenden Zeichnungen beschrieben.

Dabei sind das erste Verbindungselement 1 im Wesentlichen als Hülse und das zweite Verbindungselement 2 als Basisteil ausgebildet, das die Achsschraube einer Femur-Probeprothese in einer Nut bzw. Haltenut aufnimmt. Das zweite Verbindungselement 2 ist im Wesentlichen als länglicher Körper ausgebildet, der einen runden Querschnitt aufweist.

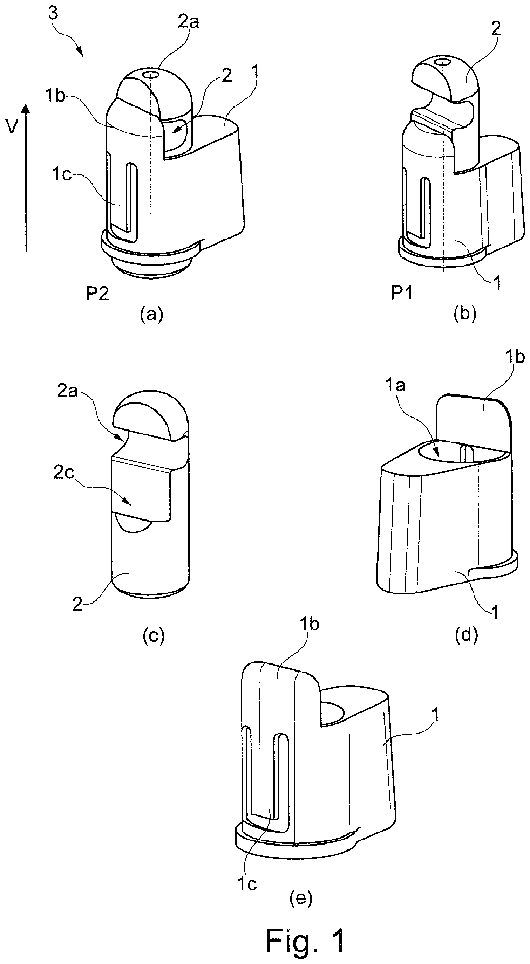

Fig. 1(a) zeigt eine Verbindungseinrichtung 3 mit einem ersten Verbindungselement 1 und einem zweiten Verbindungselement 2. Das erste Verbindungselement 1 weist ein Gegenstück 1b und eine Federlasche 1c auf. Das zweite Verbindungselement 2 weist eine Nut 2a auf. Die in Fig. 1(a) gezeigte Verbindungseinrichtung ist in der geschlossenen Position P2 bzw. das zweite Verbindungselement ist relativ zum ersten Verbindungselement in die geschlossene Position P2 verschoben. In dieser geschlossenen Position P2 ist das zweite Verbindungselement 2 zumindest abschnittsweise in das erste Verbindungselement 1 hineingeschoben und das Gegenstück 1b des ersten Verbindungselements 1 liegt der Nut 2a des zweiten Verbindungselement 2 derart verschließend gegenüber, dass ein Austreten des in der Nut 2a aufgenommenen Abschnitts IIa durch das Gegenstück 1b des ersten Verbindungselements aus der Nut 2a verhindert wird.

Fig. 1(b) zeigt die offene Position P1, in der das zweite Verbindungselement 2 zumindest abschnittsweise aus dem ersten Verbindungselement 1 herausgeschoben ist und die Nut 2a des zweiten Verbindungselements zumindest abschnittsweise derart frei liegt bzw. unbedeckt ist, dass ein aufzunehmender bzw. aufgenommener Abschnitt IIa in die Nut eingeführt bzw. aus der Nut herausgeführt werden kann.

Das zweite Verbindungselement 2 ist in dem ersten Verbindungselement 1 verschiebbar aufgenommen, sodass das zweite Verbindungselement 2 in die Verschiebungsrichtung V relativ zu dem ersten Verbindungselement 1 verschoben werden kann. Somit erfolgt der Übergang von der in Fig. 1(a) gezeigten geschlossenen Position zu der in Fig. 1(b) gezeigten offenen Position durch Verschieben des ersten bzw. zweiten Verbindungselements in die Verschiebungsrichtung V, die in den Figuren vertikal verläuft.

Fig. 1(c) zeigt das zweite Verbindungselement 2, das die Nut 2a aufweist, die senkrecht zur Verschiebungsrichtung V verläuft. Die Nut 2a definiert eine derartige Öffnung, dass sie im Wesentlichen senkrecht zur Verschiebungsrichtung V geöffnet ist. Darüber hinaus weist das zweite Verbindungselement einen Stufenabschnitt 2c auf, der in Zusammenspiel mit einem komplementären Anschlag (nicht gezeigt) des ersten Verbindungselements 1 die maximale Verschiebung des zweiten Verbindungselements 2 relativ zu dem ersten Verbindungselement 1 in der offenen Position P1 durch Anstoßen des Stufenabschnitts 2c und des Anschlags aneinander begrenzt.

Fig.1(d) zeigt ein erstes Verbindungselement mit dem Gegenstück 1b und den Innenwänden 1a, die als Führung für das zweite Verbindungselement 2 dienen. Die Innenwände 1a umschließen zumindest abschnittsweise das zweite Verbindungselement 2, das entlang den Innenwänden 1a relativ zu dem ersten Verbindungselement 1 in die Verschiebungsrichtung V verschiebbar ist.

Fig.1(e) zeigt eine andere Ansicht des ersten Verbindungselements 1 und insbesondere das Federelement 1c, das derart gegen das zweite Verbindungselement 2 drücken kann, dass ein ungewolltes Verschieben des zweiten Verbindungselements 2 in die Verschiebungsrichtung V zur offenen Position P1 hin verhindert wird.

Aus Fig. 1(e) wird des Weiteren deutlich, dass das Federelement 1c, das als Federlasche ausgebildet ist, in der Verschiebungsrichtung V zu der geschlossenen Position P2 hin angeordnet ist. Das Gegenstück 1b kann als Vorsprung ausgebildet sein, der in der Verbindungsrichtung V zu der offenen Position hin von dem ersten Verbindungselement 1 vorsteht.

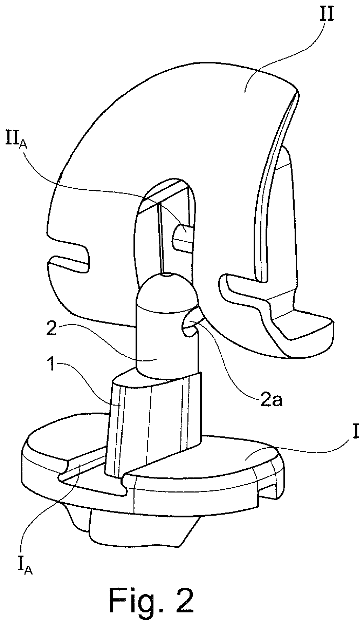

Fig. 2 zeigt zusätzlich zu der erfindungsgemäßen Verbindungseinrichtung 3 mit dem ersten Verbindungselement 1 und dem zweiten Verbindungselement 2 ein erstes Prothesenteil I, das eine Tibia-Probeprothese darstellt, und ein zweites Prothesenteil II, das eine Femur-Probeprothese darstellt. Das erste Prothesenteil I weist eine Führung Ia auf, durch die das erste Verbindungselement 1 in eine Richtung senkrecht zur Verschiebungsrichtung V geführt wird. Die Führung Ia ist als Aussparung ausgebildet, die die lösbare Aufnahme des ersten Verbindungselements 1 in dem ersten Prothesenteil I ermöglicht.

Das zweite Prothesenteil II weist einen Abschnitt IIa auf, der als Achse senkrecht zur Verschiebungsrichtung V ausgebildet ist. Diese Achse liegt zwischen zwei Kondylenabschnitten der Femur-Probeprothese. Der achsförmige Abschnitt IIa weist einen Querschnitt auf, der im Wesentlichen dem Querschnitt der Freifläche der Nut 2a entspricht.

## Patentansprüche

1. Verbindungseinrichtung (3) zur Verbindung eines ersten und eines zweiten Prothesenteils (I, II), insbesondere einer Tibia- und einer Femur-Probeprothese zur probeweisen Anordnung an den entsprechenden Knochen, wobei die Verbindungseinrichtung aufweist:
ein erstes Verbindungselement (1), das als Führung dienende Innenwände (1a) und ein Gegenstück (1b) aufweist, zur Anbringung an dem ersten Prothesenteil (I), insbesondere der Tibia-Probeprothese, und
ein zweites Verbindungselement (2), das zumindest abschnittsweise von den Innenwänden (1a) des ersten Verbindungselements umschlossen ist, entlang den Innenwänden (1a) relativ zu dem ersten Verbindungselement (1) in eine Verschiebungsrichtung (V) verschiebbar ist und eine Nut (2a) zur Aufnahme eines Abschnitts (IIa) des zweiten Prothesenteils (II), insbesondere der Femur-Probeprothese, aufweist,
wobei das zweite Verbindungselement (2) in die Verschiebungsrichtung (V) relativ zum ersten Verbindungselement (1)
in eine offene Position (P1), in der das zweite Verbindungselement zumindest abschnittweise aus dem ersten Verbindungselement herausgeschoben ist und die Nut (2a) des zweiten Verbindungselements zumindest abschnittsweise derart freiliegt, dass der Abschnitt (IIa) des zweiten Prothesenteils in die Nut eingeführt und aus der Nut herausgeführt werden kann, und
in eine geschlossene Position (P2) verschiebbar ist, in der das zweite Verbindungselement zumindest abschnittsweise in das erste Verbindungselement hineingeschoben ist und das Gegenstück (1b) des ersten Verbindungselements der Nut (2a) des zweiten Verbindungselements derart gegenüberliegt und die Nut (2a) verschließt, dass ein Austreten des in der Nut (2a) aufgenommenen Abschnitts (IIa) des zweiten Prothesenteils durch das Gegenstück (1b) des ersten Verbindungselements aus der Nut (2a) verhindert wird.

2. Verbindungseinrichtung nach Anspruch 1, bei der das Gegenstück (1b) als Vorsprung ausgebildet ist, der in der Verschiebungsrichtung (V) zu der offenen Position hin von dem ersten Verbindungselement vorsteht.

3. Verbindungseinrichtung nach einem der vorhergehenden Ansprüche, bei der die Nut (2a) eine Öffnung definiert, die im Wesentlichen senkrecht zur Verschiebungsrichtung geöffnet ist.

4. Verbindungseinrichtung nach einem der vorhergehenden Ansprüche, bei der das erste Verbindungselement ein Federelement (1c), insbesondere eine Federlasche, zum derartigen Drücken gegen das zweite Verbindungselement (2) aufweist, dass ein ungewolltes Verschieben des zweiten Verbindungselements in die Verschiebungsrichtung (V) zur offenen Position hin verhindert wird.

5. Verbindungseinrichtung nach Anspruch 4, bei der das Federelement (1c) in Bezug auf das Gegenstück (1b) in der Verschiebungsrichtung (V) zu der geschlossenen Position (P2) hin angeordnet ist.

6. Verbindungseinrichtung nach einem der vorhergehenden Ansprüche, bei der das zweite Verbindungselement (2) einen Stufenabschnitt (2c) aufweist und das erste Verbindungselement (1) einen komplementären Anschlag aufweist, sodass die maximale Verschiebung des zweiten Verbindungselements relativ zu dem ersten Verbindungselement in der offenen Position durch Anstoßen des Stufenabschnitts (2c) und des Anschlags aneinander begrenzt ist.

7. Satz mit einer Verbindungseinrichtung (3) nach einem der vorhergehenden Ansprüche, einem ersten Prothesenteil (I) und einem zweiten Prothesenteil (II), insbesondere einer Tibia- und einer Femur-Probeprothese zur probeweisen Anordnung an den entsprechenden Knochen, wobei ein Abschnitt (IIa) des zweiten Prothesenteils als Achse senkrecht zur Verschiebungsrichtung (V) und mit einem Querschnitt ausgebildet ist, der im Wesentlichen dem Querschnitt der Freifläche der Nut (2a) entspricht.

8. Satz nach Anspruch 7, bei dem das erste Prothesenteil (I) eine Führung (Ia) für das erste Verbindungselement (1) zur Anbringung des ersten Verbindungselements an dem ersten Prothesenteil in eine Richtung senkrecht zur Verschiebungsrichtung (V) aufweist.

## Claims

1. Connection device (3) for connecting a first and a second prosthesis part (I, II), in particular a tibial and a femoral trial prosthesis for trial arrangement on the corresponding bones, said connection device having:
a first connection element (1), which has inner walls (1a), serving as a guide, and a mating piece (1b), for mounting on the first prosthesis part (I), in particular the tibial trial prosthesis, and
a second connection element (2), which is enclosed at least in part by the inner walls (1a) of the first connection element, is displaceable along the inner walls (1a) relative to the first connection element (1) in a displacement direction (V) and has a groove (2a) for receiving a portion (IIa) of the second prosthesis part (II), in particular of the femoral trial prosthesis,
wherein the second connection element (2) is displaceable, in the displacement direction (V) relative to the first connection element (1),
to an open position (P1), in which the second connection element is at least in part pushed out of the first connection element, and the groove (2a) of the second connection element is at least in part exposed, such that the portion (IIa) of the second prosthesis part can be introduced into the groove and withdrawn from the groove, and
to a closed position (P2), in which the second connection element is at least in part pushed into the first connection element, and the mating piece (1b) of the first connection element lies opposite the groove (2a) of the second connection element and closes the groove (2a) in such a way that the portion (IIa) of the second prosthesis part received in the groove (2a) is prevented, by the mating piece (1b) of the first connection element, from exiting the groove (2a).

2. Connection device according to Claim 1, in which the mating piece (1b) is configured as a protrusion which, in the displacement direction (V) towards the open position, protrudes from the first connection element.

3. Connection device according to either of the preceding claims, in which the groove (2a) defines an opening which is open substantially perpendicular to the displacement direction.

4. Connection device according to one of the preceding claims, in which the first connection element has a spring element (1c), in particular a spring tab, for pressing against the second connection element (2) in such a way that an unintentional displacement of the second connection element in the displacement direction (V) towards the open position is prevented.

5. Connection device according to Claim 4, in which the spring element (1c) is arranged, with respect to the mating piece (1b), in the displacement direction (V) towards the closed position (P2).

6. Connection device according to one of the preceding claims, in which the second connection element (2) has a stepped portion (2c), and the first connection element (1) has a complementary stop, such that maximum displacement of the second connection element relative to the first connection element in the open position is limited by the stepped portion (2c) and the stop abutting against each other.

7. Kit with a connection device (3) according to one of the preceding claims, a first prosthesis part (I) and a second prosthesis part (II), in particular a tibial and a femoral trial prosthesis for trial arrangement on the corresponding bones, wherein a portion (IIa) of the second prosthesis part is configured as a shaft perpendicular to the displacement direction (V) and with a cross section corresponding substantially to the cross section of the free surface of the groove (2a).

8. Kit according to Claim 7, in which the first prosthesis part (I) has a guide (Ia) for the first connection element (1), for mounting the first connection element on the first prosthesis part in a direction perpendicular to the displacement direction (V) .

## Revendications

1. Dispositif de liaison (3) destiné à la liaison d'une première et d'une seconde parties de prothèse (I, II), en particulier d'une prothèse d'essai de tibia et d'une prothèse d'essai de fémur pour l'agencement d'essai sur les os correspondants, dans lequel le dispositif de liaison présente:
un premier élément de liaison (1), qui présente des parois intérieures servant de guide (1a) et une contre-pièce (1b), à poser sur la première partie de prothèse (I), en particulier la prothèse d'essai de tibia, et
un second élément de liaison (2), qui est entouré au moins partiellement par les parois intérieures (1a) du premier élément de liaison, qui est déplaçable le long des parois intérieures (1a) par rapport au premier élément de liaison (1) dans une direction de déplacement (V) et qui présente une rainure (2a) destinée à recevoir une partie (IIa) de la seconde partie de prothèse (II), en particulier la prothèse d'essai de fémur,
dans lequel le second élément de liaison (2) peut être déplacé dans la direction de déplacement (V) par rapport au premier élément de liaison (1)
dans une position ouverte (P1), dans laquelle le second élément de liaison est glissé au moins partiellement hors du premier élément de liaison et la rainure (2a) du second élément de liaison est au moins partiellement libre, de telle manière que la partie (IIa) de la seconde partie de prothèse puisse être introduite dans la rainure et retirée hors de la rainure, et
dans une position fermée (P2), dans laquelle le second élément de liaison est au moins partiellement glissé dans le premier élément de liaison et la contre-pièce (1b) du premier élément de liaison est disposée en face de la rainure (2a) du second élément de liaison et ferme la rainure (2a), de telle manière qu'une sortie de la partie (IIa) de la seconde partie de prothèse logée dans la rainure (2a) hors de la rainure (2a) soit empêchée par la contre-pièce (1b) du premier élément de liaison.

2. Dispositif de liaison selon la revendication 1, dans lequel la contrepièce (1b) est réalisée sous la forme d'une saillie, qui est saillante sur le premier élément de liaison dans la direction de déplacement (V) vers la position ouverte.

3. Dispositif de liaison selon l'une quelconque des revendications précédentes, dans lequel la rainure (2a) définit une ouverture, qui est ouverte essentiellement perpendiculairement à la direction de déplacement.

4. Dispositif de liaison selon l'une quelconque des revendications précédentes, dans lequel le premier élément de liaison présente un élément de ressort (1c), en particulier une patte élastique, à presser contre le second élément de liaison (2), de telle manière qu'un déplacement involontaire du second élément de liaison dans la direction de déplacement (V) vers la position ouverte soit empêché.

5. Dispositif de liaison selon la revendication 4, dans lequel l'élément de ressort (1c) est disposé par rapport à la contre-pièce (1b) dans la direction de déplacement (V) vers la position fermée (P2).

6. Dispositif de liaison selon l'une quelconque des revendications précédentes, dans lequel le second élément de liaison (2) présente une partie étagée (2c) et le premier élément de liaison (1) présente une butée complémentaire, de telle manière que le déplacement maximal du second élément de liaison par rapport au premier élément de liaison dans la position ouverte soit limité par la butée de la partie étagée (2c) et de la butée l'une sur l'autre.

7. Ensemble composé d'un dispositif de liaison (3) selon l'une quelconque des revendications précédentes, d'une première partie de prothèse (I) et d'une seconde partie de prothèse (II), en particulier d'une prothèse d'essai de tibia et d'une prothèse d'essai de fémur pour l'agencement d'essai sur les os correspondants, dans lequel une partie (IIa) de la seconde partie de prothèse est réalisée sous la forme d'un axe perpendiculaire à la direction de déplacement (V) et avec une section transversale, qui correspond essentiellement à la section transversale de la face libre de la rainure (2a).

8. Ensemble selon la revendication 7, dans lequel la première partie de prothèse (I) présente un guide (Ia) pour le premier élément de liaison (1) pour la pose du premier élément de liaison sur la première partie de prothèse dans une direction perpendiculaire à la direction de déplacement (V).
